# EUROPEAN PATENT SPECIFICATION

(11) **EP 4 021 366 B1**
(45) Date of publication and mention of the grant of the patent: **09.04.2025**
(21) Application number: 20885473.7
(22) Date of filing: 23.10.2020
(51) Int. Cl.: A61G 7/057, A61N 1/20, A61N 1/36, A61B 5/00, A61B 5/103, A61B 5/11, G16H 20/30, A61N 1/04

(54) **ELECTRICAL STIMULATION SYSTEM**
ELEKTRISCHES STIMULATIONSSYSTEM
SYSTÈME DE STIMULATION ÉLECTRIQUE

(30) Priority: 04.11.2019 TR 201917004
(43) Date of publication of application: 06.07.2022
(73) Proprietor: Zorluteks Tekstil Ticaret Ve Sanayi Anonim Sirketi, 34394 Sisli/Istanbul (TR)
(72) Inventor: YILDIRIM, Murat, Lüleburgaz/Kirklareli (TR); BAYRAKTAR, Özge, Lüleburgaz/Kirklareli (TR)
(74) Representative: Yamankaradeniz, Kemal
(86) International application number: PCT/TR2020/050984
(87) International publication number: WO 2021/091508

(56) References cited:
- WO-A1-2009/046366
- WO-A1-2014/186894
- WO-A1-2014/186894
- WO-A1-2018/098417
- WO-A2-2010/119441
- CN-U- 206 910 326
- FR-A1- 2 833 497
- US-A1- 2016 186 366
- US-A1- 2017 106 188
- US-A1- 2017 156 662
- US-A1- 2017 156 662
- US-A1- 2019 053 761
- US-A1- 2019 240 475

## Description

### Technical Field

Invention relates to an electrical stimulation system covering home technical textiles, medical and hygiene technical textile group.

### Present State of the Art

Local tissue ulcers which are an essential health problem in Turkey and worldwide due to high treatment costs and developed as a result of exposure of tissues in bone extension zones of body to pressure for long time are called "pressure ulcers". Occurrence of pressure ulcers is because of long-time or excessive exposure of any part of body to pressure. In daily life standing, lying down, sitting and moving which are various postural changes are physiological protective mechanisms of our body. However, pressure ulcers occur due to body spasticity due to becoming bedridden by patients suffering from sense loss or unconsciousness.

Among current applications regarding prevention, care and treatment of pressure ulcers are studies developed by National Pressure Ulcer Advisory Panel (NPUAP). This study comprises information about change of patient positions of those bedridden individuals at least once every 2 hours, placement onto mattress/chair pillow, use of pillow or foam to prevent direct contact of bone areas such as knees and elbows, use of tools totally eliminating pressures in heels, prevention of press injuries to family members and caregivers.

Patients that hospitalized in intensive care units or inpatient treatment units in hospitals and bedridden patients at home is not possible provide their own body movement without assistance. For that reason, health workers at hospitals and the care givers giving cares to such patients at home have big responsibility. It is tried to reduce body pressure points by means of change lying position of patient once every two hours. In some cases, position change may not be adequate to prevent pressure wounds.

Various supportive surfaces have been designed to prevent development of pressure ulcers and reduce pressure on tissues due to cost of changing patient's position frequently and limitations. Among products available in the market are several products such as fixed compressed air beds, changing compressed air beds, foam body supportive products. However, these products can not eliminate pressure ulcers completely. This is because pressure is continuous in such systems despite reduction in the pressure, therefore it causes some distortions in blood circulation in time and pressure ulcers develop.

Among present applications are physical treatment applications other than pressure reducing products are addressed in the treatment of pressure ulcers. Among such applications are laser, ultrasonic, ultraviolet, electrical stimulation, hydrotherapy. Only electrical stimulation is authorized to be recommended by AHCPR (Agency for Health Care Policy and Research). In literature, existing electrical stimulation applications are in specified dosages onto wound areas at routine intervals. In general, after wound is totally healed, electrical stimulation treatment is ended. Ultraviolet, low energy laser, hydrotherapy and ultrasound are not proven level to be recommended for treatment.

Although several applications have been developed to prevent and heal pressure ulcers, pressure ulcers affect care cost and life quality and continue to be one of important health issues in patient care institutes. Treatment process of pressure ulcers is considerably hard and economically big burden since starting and life quality of patient decreases and patient close relatives' care burden increases. Therefore, current supportive surfaces are inadequate in this matter.

In the related art, patent application numbered TR2017/10856 discloses a smart mat model preventing development of pressure ulcers on body skin of bedridden patients due to long time under pressure. The mat disclosed under said document does not comprise textile base sensor and electrical stimulation application disclosed under this patent description.

In the related art, patent application numbered US20160186366A1 discloses a method for making a textile sensor and the textile sensor can include selecting a combination of variables from the group consisting of yarn variables, stitch variables, and textile variables. However, there is no information in the invention about the transmission of electric current to the tissue in order to nourish the tissue in immobilized body parts and to prevent the development of ulcers in the patient.

In the related art, patent application numbered WO2018098417A1 discloses systems and methods are described for monitoring, treating, and preventing a pain state of an individual. However, there is no information in the invention about the transmission of electric current to the tissue in order to nourish the tissue in immobilized body parts and to prevent the development of ulcers in the patient. Furthermore, the invention does not mention a portable system. In addition, the invention uses adhesive, which is uncomfortable for the patient, and the invention does not involve stimulation of a specific area.

In the related art, patent application numbered US20170156662A1 discloses systems and methods are described for monitoring an individual subject and facilitating a motion regimen of the individual subject. However, there is no information in the invention about the transmission of electric current to the tissue in order to nourish the tissue in immobilized body parts and to prevent the development of ulcers in the patient. In addition, this invention works with accelerometer and adhesive patches make the patient uncomfortable.

In the related art, patent application numbered US20170106188A1 discloses an apparatus includes multiple first reservoirs and multiple second reservoirs joined with a substrate. However, there is no information in the invention about the transmission of electric current to the tissue in order to nourish the tissue in immobilized body parts and to prevent the development of ulcers in the patient. On the other hand, there is no information in the invention about stimulating only certain tissues when inactivity is detected for a certain period of time.

In the related art, patent application numbered WO2014186894A1 discloses method and assembly for acquiring pressure data. A pressure sensor is applied to a target surface on an individual. However, there is no information in the invention about the transmission of electric current to the tissue in order to nourish the tissue in immobilized body parts and to prevent the development of ulcers in the patient.

Patent application document numbered US9700717B2 of the related art discloses an instrument detecting conditions eligible for pressure ulcer development and reversing such conditions. The invention described under said document discloses a product similar to underwear that can be worn and in contact with human skin. The underwear of document numbered US9700717B2 can be worn while patient is in bed or on wheelchair. Therefore, it does not mention "adaptation to bed or wheelchair". Invention disclosed under this description comprises a bed pad. Said bed pad eliminates need for contact with human skin.

The document numbered US9700717B2 is an embodiment limited onto people having SCI (Spinal Cord Injury) diseases. Although the term other areas open to pressure ulcer development tries to broad the scope, whole document contain explanations of design for tailbone and spinal zone there around and treatment thereof. Said document particularly aims at T5 and T12 vertebras in tail end. That is, the invention disclosed under said document is a design limitation from wearing of underwear.

Document numbered US9700717B2 uses HVPGS (High voltage Pulse galvanic stimulation) as electrical stimulation and gives details thereof. Invention disclosed under this description does not suggest any limitation regarding electrical stimulation. In literature, it is shown that electrical stimulation (ES) is effective in wound healing. Various applications and electrical stimulation methods including direct current (DC), alternate current (AC), high voltage pulse current (HVPC), low intensified direct current (LIDC) and electro biological recycling electrical stimulation are described.

Document numbered US9700717B2 uses mostly tail end parameters to monitor pressure ulcer development. The invention disclosed under this description aims to start monitoring and stimulation system automatically after a fixed time period lapse subject to period spent by patient in bed.

Document numbered US9700717B2 focuses on monitoring and prevention system for pressure ulcer development in T5 and T12 vertebras Therefore, there is no invention design for monitoring and prevention of pressure ulcer development in other areas of body. However, in the invention disclosed under this description, all body areas are targeted, each body area is monitored separately and separate stimulation is applied. For instance, if pressure change in shoulder area is not observed and pressure change is observed in tail end area, only body area open to development of pressure ulcers, which is shoulder area in this case, is stimulated by stimulation in a selected manner, tail end area is not stimulated. As a result, due to above described disadvantages and inadequacy of existing solutions, it has been necessary to make development in the related art.

### Purpose of the Invention

The invention is defined in claim 1. Further aspects and preferred embodiments are defined in the appended claims. Aspects, embodiments and examples of the present disclosure which do not fall under the scope of the appended claims do not form part of the invention and are merely provided for illustrative purposes.

The invention has been developed with inspiration from existing situation and aims to eliminate the above mentioned disadvantages.

Purpose of this invention is achieve an electric simulation system covering home technical textiles, medical and hygiene technical textile group.

Another purpose of the invention is to prevent "pressure ulcers" with a proactive approach.

A further purpose of the invention is to provide a selective electrical stimulation application for certain areas of body by perceiving change in body pressure of patient on bed.

Another purpose of the invention is to provide an effect equivalent to natural movement mechanism of body by stimulating muscles surrounding blood vessels.

A further purpose of the invention is to provide that blood pressure starting to lose order under stable pressure of body gains its order again and that oxygen and nutrition needs of cells are not interrupted.

Another purpose of the invention is to provide removal of waste products from cells without allowing their accumulation.

A further purpose of the invention is to keep defence mechanisms active against invasions of cells by micro-organisms.

The structural and characteristics features of the invention and all advantages will be understood better in detailed descriptions with the figures given below and with reference to the figures, and therefore, the assessment should be made taking into account the said figures and detailed explanations.

### Brief Description of the Drawings

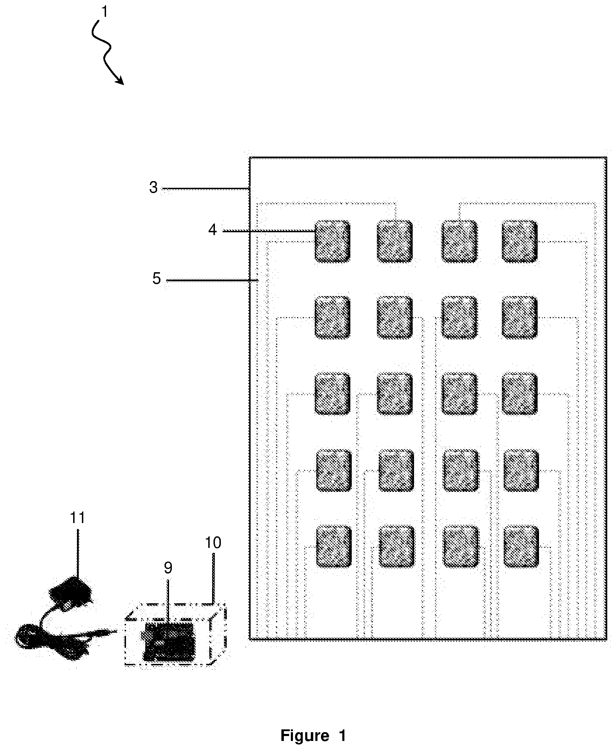

Figure 1 is an illustrative schematic view of system of the invention.

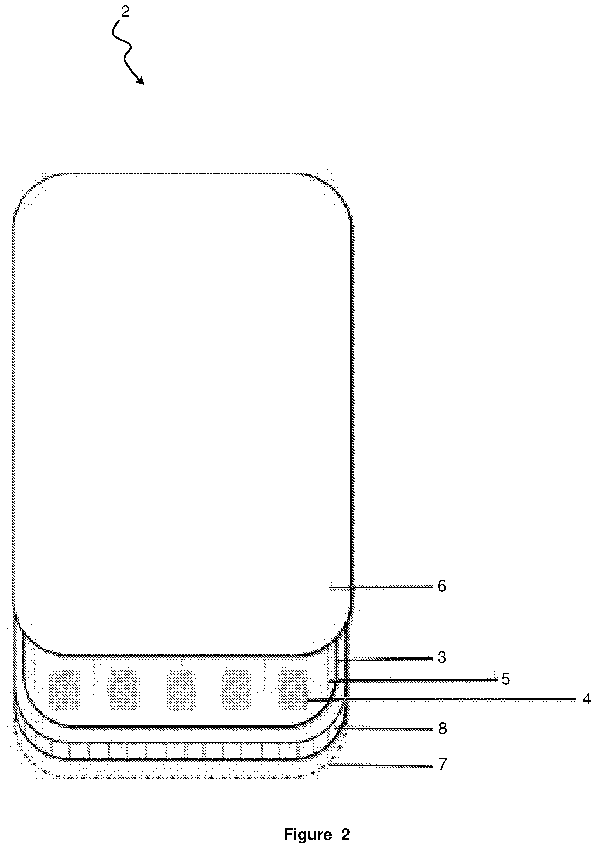

Figure 2 is an illustrative view of bed pad of the system of the invention.

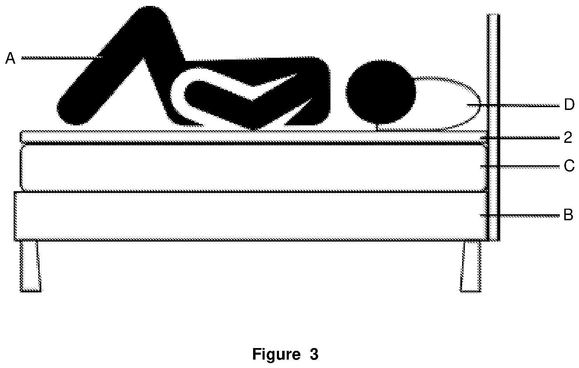

Figure 3 is an illustrative view showing use of bed pad of the system of the invention in the bedstead/bed base, bed and pillow.

### Description of Part References

1. Electrical stimulation system
2. Bed pad
3. Insulation fabric
4. Electronic textile apparatus
5. Conductive yarns
6. Upper fabric
7. Lower fabric
8. Visco/sponge
9. Control unit
10. Equipment box
11. Adapter
   A. Patient
   B. Bedstead/bed base
   C. Bed
   D. Pillow

### Detailed Description of the Invention

In this detailed description, the preferred embodiments of an electrical stimulation system (1) being subject of the invention have been described only for purpose of better understanding of the matter.

Invention relates to an electric simulation system (1) covering home technical textiles, medical and hygiene technical textile group.

The system (1) disclosed under the invention is a unique selective embodiment applying electrical stimulation in order to prevent pressure ulcers with a proactive approach. Thus a proactive solution is provided for prevention of pressure ulcer development where existing applications are inadequate.

With the system (1) disclosed under the invention, differently from existing electrical simulation applications in the literature, increasing blood pressure at body stable pressure points before development of pressure ulcers. In literature, existing electrical stimulation applications are in specified dosages onto wound areas at routine intervals. In general, after wound is totally healed, electrical stimulation treatment is ended. In the system (1) disclosed under the invention, a selective mechanism is developed instead of applying a fixed electrical stimulation to whole body. Body pressure distribution of patients (A) onto the bed is constantly measured for this purpose. With measurement, long-time fixed body pressure points are identified and electrical impulse is given to only such area (areas). In the system (1) electrical stimulation is applied, muscles surrounding blood vessels are stimulated and an effect equivalent to natural movement mechanism of body is provided. Thus blood flow starting to become disorder under stable pressure of body is put into order and suspension of oxygen and nutrition is prevented, accumulation of waste products in cells is provided and defence mechanism of cells is kept active against invasion of cells by micro-organisms, and thus pressure ulcers are proactively prevented.

The system (1) disclosed under the invention is preferably used in environment where bedstead/bed base (B), bed (C) put onto said bedstead/bed base (B) and pillow (D) are used.

The system (1) of the invention comprises;
❖ A bed pad (2) comprises,
   ∘ with a proactive approach, being unique for prevention of "pressure ulcers" an important health problem with high treatment costs and defined as local tissue injuries developing as a result of long time exposure of tissues in bone extension areas of body to pressure,
   ∘ used for the purpose of increasing blood pressure at stable pressure points of body before development of wound yet,
   ∘ Realizing a selective operation for related area instead of applying fixed electrical stimulation to whole body;
   ∘ having conductive textile materials,
   o measuring body pressure distribution of patients (A) onto the bed,
   o With measurement, identifying long-time fixed body pressure points and giving electrical impulse to only such area(areas),
   o providing an effect equivalent to natural movement mechanism of body by applying electrical stimulation, stimulating muscles surrounding blood vessels and
   o thus putting blood flow starting to become disorder under stable pressure of body into order and preventing suspension of oxygen and nutrition, accumulation of waste products in cells and keeping defence mechanism of cells active against invasion of cells by micro-organisms, and thus providing proactive prevention of pressure ulcers,

Bed pad (2) comprises,
❖ insulation fabric (3) having insulation feature and placed inside bed pad (2),
❖ electronic textile apparatus (4) placed on insulation fabric (3) measuring body pressure change of patient (A) by use of capacitive sensor function and applying current (mA) / vibration (Hz) to long-time body pressure points identified as a result of measurements, that is, pressure areas by means of electrical stimulation function,
❖ Conductive yarns (5) located onto insulation fabric (3), placed between electronic textile apparatus (4), providing formation of electronic textile structure and thus obtaining capacitive pressure sensor and electrical stimulation surface,
❖ upper fabric (6) covering insulation fabric (3) and thus forming upper layer, and
❖ lower fabric (7) covering beneath of insulation fabric (3) and thus forming lower layer.

### (Figure 2).

In the preferred embodiment of the invention, bed pad (2) comprises, visco/sponge (8) placed under insulation fabric (3), supporting body and providing contribution to balance pressure.

The system (1) of the invention comprises;
❖ control unit (9) connected to electronic textile apparatus (4) electrically by means of conductive yarns (5) and having software developed for performance of measurement of capacitive pressure of electronic textile apparatus (4) and performance of electrical stimulation functions,
❖ equipment box (10) housing control unit (9) and
❖ adapter (11) supplying energy required for operation of control unit (9).

### (Figure 1)

Bedridden (C) patients lie down motionless on a bed (C) located onto bed base/bedstead (B) generally in hospital or home. Textiles such as aleze, sheet etc. can also be used on bed (C). Home textiles such as pillow (D) product to support patient's (A) head and pillow cover put onto the product can be used on the bed (C).

Bed pad (2) provided in the system (1) of the invention is placed on the bed (C). If needed, pillow (D) product can be placed on bed pad (2). When desired, home textile products such as bed sheet can also be used on the bed pad (2).

Bed pad (2) comprises upper fabric (6), electronic textile apparatus (4) displaying capacitive / electrical stimulation function processed by use of conductive yarns (5) on the insulation fabric (3), integration of visco/sponge (8) and lower fabric (7) layers. Use of visco/sponge (8) layer is believed to be beneficial to integrate it into bed pad (2) as it provides contribution for balancing body pressure on bed and supporting patient's (A) spine.

Insulation fabric (3) whereon conductive yarns (5) are made in certain patters becomes electronic textile apparatus (4) having capacitive pressure measurement sensor and electrical stimulation function. Sensor embodiments allocated to multiple different areas to represent body areas open to pressure ulcer development can be formed by conductive yarns (5). Thus instead of applying electrical stimulation application to whole body, local application is possible. A unique software is developed to perform capacitive pressure measurement of electronic textile apparatus (4) and electrical stimulation functions. The software is embedded into control unit (9). Connection is provided by use of conductive yarns (5) between control unit (9) and electronic textile apparatus (4). Control unit (9) is provided inside equipment box (10). Electricity needed for operation of control unit (9) is supplied by adapter (11).

The system (1) of the invention measures pressure change by capacitive perception in order to proactively prevent risk of pressure ulcer development occurring as a result of patient's (A) lying on bed pad (2) in same position for a time period longer than preset threshold value. In case of any failure in performance of duties by person responsible for changing position of patient (A) during time period over threshold period, electrical stimulation application is made to the body area of patient (A) where pressure change has not occurred. In this operation, electronic textile apparatus (4) is activated by software provided in control unit (9) and electronic textile apparatus (4) provides submission of electrical impulse. Thus pressure distribution on area of patient (A) remaining motionless is balanced, blood circulation rate of patient (A) become normal and no suspension in oxygenizing of patient's (A) tissue occur. Thus work load on the persons responsible for caring patient (A) decreases and occurrence of pressure ulcers arising from negligence is reduced.

## Claims

1. An electrical stimulation system (1) increasing the blood circulation at the stationary pressure points of the body before the patient's (A) pressure ulcer has yet formed, comprising a bed pad (2):
• having an insulating fabric (3) integrating bedstead/bed base (B),
• and having a control unit (9), ,
• having conductive textile materials,
• measuring body pressure distribution of patients (A) onto the bed (C),
• with measurement, identifying long-time fixed body pressure points and giving electrical impulse to only such area(s),
and **characterized by** comprising:
❖ an electronic textile apparatus (4) placed on the insulation fabric (3), which is configured to measure body pressure change of a patient (A) by use of capacitive sensor function and which is configured to apply current (mA) / vibration (Hz) to long-time body pressure points/areas identified as a result of said measurements by means of electrical stimulation function,
❖ conductive yarns (5) located onto the insulation fabric (3), placed between the electronic textile apparatus (4), configured to provide formation of electronic textile strcture and configured to obtain capacitive pressure sensor and electrical stimulation surface,
❖ upper fabric (6) covering insulation fabric (3) and thus forming upper layer, and
❖ lower fabric (7) covering beneath of insulation fabric (3) and thus forming lower layer; and
wherein the bed pad (2) comprises a visco/sponge (8) placed under the insulation fabric (3) supporting the body and providing contribution to balancing pressure.

## Patentansprüche

1. Elektrisches Stimulationssystem (1), das die Blutzirkulation an den stationären Druckpunkten des Körpers erhöht, bevor sich das Druckgeschwür des Patienten (A) gebildet hat, umfassend eine Bettauflage (2):
• die einen isolierenden Stoff (3) aufweist, der Bettgestell/Bettkasten (B) integriert,
• und eine Bedieneinheit (9) aufweist,
• die leitende textile Materialien aufweist,
• die die Körperdruckverteilung von Patienten (A) auf dem Bett (C) misst,
• mit Messung, Identifizieren von seit langem fixierten Körperdruckpunkten und Abgeben von elektrischen Impulsen an nur solche Bereiche,
und **dadurch gekennzeichnet, dass** es Folgendes umfasst:
❖ eine elektronische textile Vorrichtung (4), die auf dem Isolierstoff (3) platziert ist, die konfiguriert ist, um eine Körperdruckänderung eines Patienten (A) unter Verwendung einer kapazitiven Sensorfunktion zu messen, und die konfiguriert ist, um Strom (mA) / Vibration (Hz) an Langzeit-Körperdruckpunkte/-bereiche anzulegen, die als Resultat der Messungen mittels einer elektrischen Stimulationsfunktion identifiziert werden,
❖ leitende Fäden (5), die sich auf dem Isolierstoff (3) befinden, zwischen der elektronischen textilen Vorrichtung (4) angeordnet und konfiguriert sind, um eine elektronische textile Struktur zu bilden und konfiguriert sind, um einen kapazitiven Drucksensor und eine elektrische Stimulationsfläche zu erlangen,
❖ einen oberen Stoff (6), der den Isolierstoff (3) bedeckt und somit die obere Schicht bildet, und
❖ einen unteren Stoff (7), der die Unterseite des Isolierstoffs (3) bedeckt und somit die untere Schicht bildet; und
wobei die Bettauflage (2) einen Visko-Schwamm (8) umfasst, der unter dem Isolierstoff (3) platziert ist, den Körper stützt und einen Beitrag zum Druckausgleich bereitstellt.

## Revendications

1. Système de stimulation électrique (1) augmentant la circulation sanguine au niveau des points de pression stationnaires du corps avant que la plaie de pression du patient (A) ne se soit formée, comprenant un surmatelas (2) :
• comportant un tissu isolant (3) intégrant un châlit/sommier (B),
• et comportant une unité de commande (9),
• comportant des matériaux textiles conducteurs,
• mesurant la répartition de la pression corporelle des patients (A) sur le lit (C),
• avec une mesure, identifiant les points de pression corporelle fixes de longue durée et donnant une impulsion électrique uniquement à cette ou ces zones,
et **caractérisée** en comprenant :
❖ un appareil textile électronique (4) placé sur le tissu isolant (3), qui est configuré pour mesurer le changement de pression corporelle d'un patient (A) à l'aide d'une fonction de capteur capacitif et qui est configuré pour appliquer un courant (mA)/une vibration (Hz) à des points/zones de pression corporelle de longue durée identifiés à la suite desdites mesures au moyen d'une fonction de stimulation électrique,
❖ des fils conducteurs (5) situés sur le tissu isolant (3), placés entre l'appareil textile électronique (4), configurés pour assurer la formation d'une structure textile électronique et configurés pour obtenir un capteur de pression capacitif et une surface de stimulation électrique,
❖ un tissu supérieur (6) recouvrant le tissu isolant (3) et formant ainsi une couche supérieure, et
❖ un tissu inférieur (7) recouvrant le dessous du tissu isolant (3) et formant ainsi une couche inférieure ; et
ledit surmatelas (2) comprenant une visco/éponge (8) placée sous le tissu isolant (3) supportant le corps et contribuant à équilibrer la pression.
